# DEMANDE DE BREVET EUROPEEN

(11) **EP 1 099 429 A1**
(43) Date de publication de la demande: **16.05.2001**
(21) Numéro de dépôt: 99811028.2
(22) Date de dépôt: 09.11.1999
(51) Int. Cl.: A61F 2/44

(54) **Cage de fusion intersomatique comportant un bec de canard**

(71) Demandeur: Laciter Management Limited, 3724 Limassol (CY)
(72) Inventeur: Vryonides, Stathis, Laciter Management Ltd., P.O. Box 4425, 3724 Limassol (CY)
(74) Mandataire: Moinas, Michel

(57) **Abrégé**

Cage de fusion intersomatique s'étendant suivant une direction longitudinale (D) pour être insérée entre deux corps vertébraux par des impacts exercés sur une extrémité (7).

Selon l'invention, l'extrémité opposée (2) suivant la direction longitudinale (D) est conformée en bec de canard (6, 8, 9) pour faciliter l'insertion entre les corps vertébraux. Le bec de canard comprend une lèvre commune (6) réunissant deux échancrures (8, 9) formées dans deux faces d'appui opposées (4, 5) de la cage.

De préférence, le bec de canard s'étend sur une fraction de longueur (I) comprise entre un cinquième et un quart de la longueur (L) totale de la cage, et la lèvre (6) possède une hauteur (h) qui est comprise entre un quart et un tiers de la hauteur (H) entre les faces d'appui opposées (4, 5).

Les faces d'appui opposées (4, 5) possèdent en surface un motif de rugosité (21) en damier réalisé par faisceau laser.

## Description

L'invention se rapporte à une cage de fusion intersomatique, et en particulier à une cage qui s'étend suivant une direction longitudinale (D) pour être insérée entre deux corps vertébraux par des impacts exercés sur une extrémité (7) de la cage.

La cage est insérée entre les corps vertébraux adjacents pour restaurer chez un patient une hauteur et une statique convenable d'un espace discal, en particulier de la région lombaire de la colonne vertébrale.

On connaît du document FR 2 762 779 une cage de fusion intersomatique s'étendant suivant une direction longitudinale en ayant une forme globalement parallélépipédique. Les parois latérales de la cage sont plates pour réduire l'encombrement général de la cage et permettre d'insérer deux cages dans l'espace discal afin d'augmenter la stabilité des deux corps vertébraux.

La cage s'intercale entre les corps vertébraux par deux surfaces d'appui opposées et convexes l'une et l'autre pour suivre la configuration naturellement concave de l'espace discal. Par ce moyen, on augmente la surface de contact entre la cage et les deux corps vertébraux et on assure un écartement plus stable de l'espace discal.

La cage est percée d'ouvertures pour permettre à un greffon osseux disposé à l'intérieur de fusionner avec les disques vertébraux. Dans l'exemple décrit dans le document précité, les faces d'appui opposées et convexes possèdent une ouverture oblongue qui traverse la cage perpendiculairement à la direction longitudinale pour créer une communication entre les deux corps vertébraux. Cette ouverture permet au greffon de créer un pont osseux entre les corps vertébraux, l'écartement discal anatomique du patient étant restauré par la cage. Les parois latérales plates possèdent également des ouvertures débouchant sur l'ouverture oblongue pour favoriser l'irrigation sanguine du greffon osseux. Ces ouvertures contribuent aussi à renforcer le pont osseux entre les corps vertébraux.

La cage est insérée entre les corps vertébraux en appliquant des impacts sur une des ses extrémités. Toutefois, et malgré la forme convexe des faces d'appui opposées, l'impaction peut conduire à des cassures de l'un ou de l'autre des corps vertébraux, et particulièrement des rebords osseux antérieurs de ces derniers lorsque, comme habituellement, la cage est insérée par voie postérieure.

On observe de plus que certaines cages après insertion ont tendance à se déplacer sous l'action des corps vertébraux, particulièrement en sens opposé à l'insertion.

Le but de l'invention est d'apporter une amélioration aux cages existantes en palliant les inconvénients décrits précédemment.

A cet effet, l'invention a pour objet une cage de fusion intersomatique s'étendant suivant une direction longitudinale pour être insérée entre deux corps vertébraux par des impacts exercés sur une extrémité, caractérisée en ce que l'extrémité opposée suivant la direction longitudinale est conformée en nez ou bec de canard pour faciliter l'insertion entre les corps vertébraux.

Lors de l'insertion de la cage, l'extrémité conformée en nez ou bec de canard pénètre facilement dans l'espace discal et écarte progressivement les deux corps vertébraux adjacents. De cette façon, on réduit considérablement le risque de cassure des corps vertébraux au moment de l'impaction.

De plus, il a été constaté que le déplacement de la cage en sens opposé à l'insertion était dû à l'action des rebords osseux antérieurs des corps vertébraux et des mouvements relatifs des corps vertébraux adjacents, particulièrement dans la phase postopératoire où la fusion intersomatique n'est pas encore réalisée. Selon l'invention, le bec de canard permet à l'extrémité de la cage de se placer entre les rebords osseux antérieurs. De cette façon, les efforts qu'ils exercent sur la cage n'ont plus tendance à la déplacer en sens opposé à l'insertion.

Selon un mode particulier de réalisation de l'invention, le bec de canard comprend une lèvre commune réunissant deux échancrures formées dans les deux faces d'appui opposées de la cage. Ce mode de réalisation permet avantageusement de former par usinage les échancrures dans des faces d'appui initialement non échancrées. Il est toutefois possible de réaliser les faces d'appui et les échancrures en une seule opération, par exemple par moulage.

De préférence, le bec de canard s'étend sur une fraction de longueur comprise entre un cinquième et un quart de la longueur totale de la cage. Cet intervalle prend en compte la dispersion statistique chez l'homme des rebords osseux dans les corps vertébraux.

De préférence également, et pour les mêmes raisons, la lèvre du bec de canard possède une hauteur qui est comprise entre un quart et un tiers de la hauteur entre les faces d'appui opposées de la cage.

Dans le document précité, il est prévu que la cage possède des angles externes arrondis pour éviter la lésion éventuelle des racines nerveuses et d'autres tissus mous. Selon un mode préféré de réalisation de la présente invention, les angles de la cage sont non seulement arrondis mais cassés en biseau. Cette forme particulière diminue encore le risque de traumatisme des racines nerveuses lors du retrait de la cage.

D'autres avantages de l'invention sont mis en relief avec la description détaillée de l'invention illustrée par les dessins.
- La figure 1 est une vue schématique en perspective de la cage intersomatique.
- La figure 2 est une vue en plan de dessus de la cage.
- La figure 3 est une vue en plan de côté de la cage.
- La figure 4 est une vue en coupe partielle d'une extrémité de la cage sur laquelle on exerce des impacts lors de l'insertion.
- La figure 5 est une vue de côté montrant de façon schématique la cage des figures 1 à 4 en position intercalée entre deux corps vertébraux.

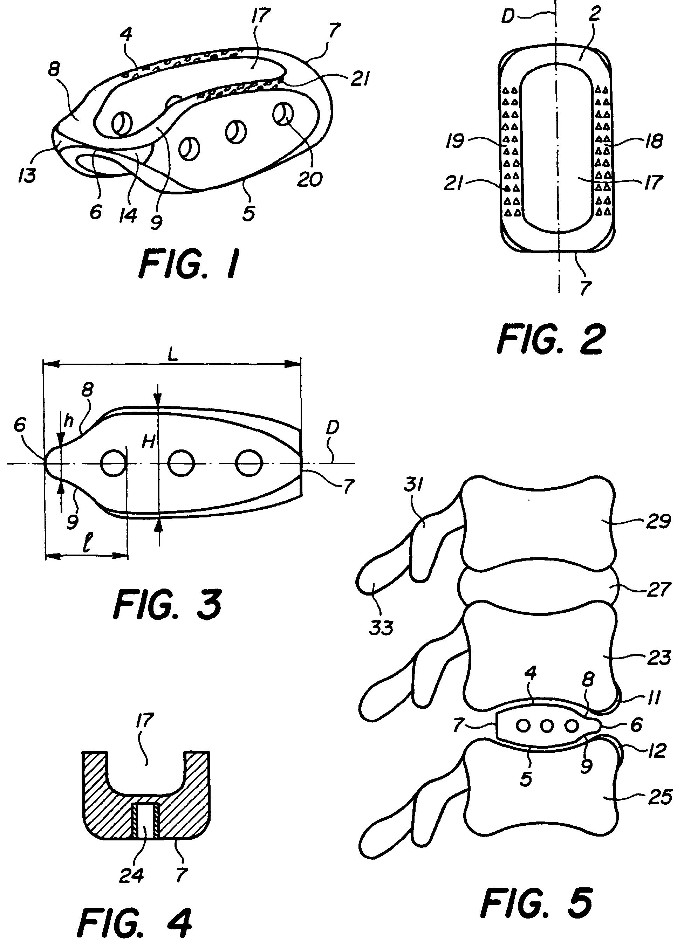

Une cage de fusion intersomatique s'étend, figures 1 à 3, suivant une direction longitudinale D pour être insérée entre deux corps vertébraux lors d'une chirurgie rachidienne chez l'homme, et en particulier de la région lombaire de la colonne vertébrale.

La cage s'inscrit globalement dans un parallélépipède rectangle. Par mesure de simplification, les qualificatifs supérieur, inférieur, latéral, antérieur et postérieur seront utilisés par analogie avec les six faces dudit parallélépipède rectangle, lorsque la cage est en position dans l'espace intervertébral, comme cela est illustré par la figure 5. Sur cette figure, la partie à droite représente la face antérieure, tandis que la partie à gauche représente la face postérieure. La cage est intercalée entre deux corps vertébraux 23 et 25. Pour augmenter la stabilité des corps vertébraux, il est prévu d'insérer deux cages parallèlement l'une par rapport à l'autre dans le même espace discal. On a également représenté sur la figure 5 un disque intervertébral sain 27 entre deux corps vertébraux 23 et 29. De façon schématique, la figure 5 illustre chaque corps vertébral avec un pédicule 31 et une épineuse 33.

La cage comprend deux parois latérales 18 et 19 plates et s'intercale entre les deux corps vertébraux 23 et 25 par deux surfaces d'appui supérieure 4 et inférieure 5 opposées et convexes l'une et l'autre pour suivre la configuration naturellement concave de l'espace discal. Des ouvertures 17 et 20 sont prévues pour permettre à un greffon osseux disposé à l'intérieur de fusionner avec les disques vertébraux. Dans l'exemple qui est décrit, les faces d'appui 4 et 5 opposées et convexes possèdent une ouverture oblongue ou ovoïde 17 qui traverse la cage perpendiculairement à la direction longitudinale D pour créer une communication entre les deux corps vertébraux. Les parois latérales plates 18 et 19 possèdent également des ouvertures 20 débouchant sur l'ouverture oblongue pour favoriser l'irrigation sanguine du greffon osseux.

La cage est insérée entre les corps vertébraux en appliquant des impacts sur une extrémité postérieure 7.

Selon l'invention, l'extrémité antérieure 2, opposée suivant la direction longitudinale D à l'extrémité postérieure 7, est conformée en nez ou bec de canard 6, 8 et 9 pour faciliter l'insertion de la cage entre les corps vertébraux 23 et 25.

Dans l'exemple d'illustration de l'invention, visible sur les figures 1 et 3, le bec de canard comprend une lèvre commune 6 réunissant des échancrures 8 et 9 formées dans les deux faces d'appui supérieure et inférieure 4 et 5. La lèvre 6 a également un profil convexe et possède avantageusement des angles cassés en biseau 13 et 14.

Comme indiqué précédemment, lors de l'insertion de la cage, l'extrémité conformée en bec de canard pénètre facilement dans l'espace discal et écarte progressivement les deux corps vertébraux adjacents sans les heurter à angle vif. De cette façon, on réduit considérablement le risque de cassure des corps vertébraux au moment de l'impaction.

De plus, le bec de canard permet à l'extrémité de la cage de se placer entre les rebords osseux antérieurs 11 et 12 des corps vertébraux. De cette façon, les efforts qu'ils exercent sur la cage n'ont plus tendance à la déplacer en sens opposé à l'insertion.

Autrement dit, lorsque la cage intersomatique est mise en place dans l'espace intervertébral, les rebords osseux 11 et 12 que l'on rencontre sur les faces internes et antérieures des corps vertébraux viennent se loger de part et d'autre au niveau des échancrures 8 et 9 du bec de canard. La configuration de l'extrémité de la cage permet donc d'avoir un appui anatomique des faces supérieure et inférieure 4 et 5 sur les parois internes des corps vertébraux 23 et 25 sans créer de gêne au niveau des rebords osseux 11 et 12.

De préférence, comme cela est visible sur la figure 3, la hauteur h de la lèvre 6 du bec de canard est comprise entre 1/4 et 1/3 de la hauteur H mesurée entre les faces d'appui supérieure 4 et inférieure 5 de la cage.

Dans le mode de réalisation illustré par les figures, le bec de canard s'étend, suivant la direction longitudinale D, sur une longueur I compris entre 1/5 et 1/4 de la longueur totale L de la cage mesurée entre les extrémités antérieure 2 et postérieure 7.

De façon avantageuse, les faces d'appuis 4 et 5 ont des angles 16 cassés en biseau du côté de l'extrémité postérieure 7. De cette façon, on évite de traumatiser les racines nerveuses lors du retrait de la cage.

L'insertion de la cage dans l'espace intervertébral est communément réalisée grâce à un outil d'impaction en prise avec l'extrémité postérieure 7.

Avantageusement, comme visible sur la figure 4, un trou taraudé 24 est prévu pour recevoir une tige filetée. On préfère un trou borne ou sans lumière, c'est-à-dire un trou qui ne débouche pas dans l'ouverture oblongue 17, pour empêcher la migration de fragments osseux à travers le trou taraudé pouvant entraîner une compression des racines nerveuses. 9. Lors de l'insertion, on exerce des impacts sur une tige filetée vissée dans le trou taraudé 24 de l'extrémité 7 opposée à l'extrémité 2 conformée en bec de canard.

La cage selon l'invention est réalisée dans un matériau biocompatible, comme par exemple un alliage de titane. Comme indiqué précédemment, les échancrures peuvent être formées par usinage des faces d'appui initialement non échancrées, ou par un moulage simultané des faces et des échancrures ainsi que de la lèvre commune.

D'une façon avantageuse, les faces d'appui opposées 4, 5 possèdent en surface un motif de rugosité 21 pour favoriser l'accrochage avec les corps vertébraux. On préfère un motif en stries, avantageusement en damier, réalisé par faisceau laser.

## Revendications

1. Cage de fusion intersomatique s'étendant suivant une direction longitudinale (D) pour être insérée entre deux corps vertébraux (23, 25) par des impacts exercés sur une extrémité (7), caractérisée en ce que l'extrémité opposée (2) suivant la direction longitudinale (D) est conformée en bec de canard (6, 8, 9) pour faciliter l'insertion entre les corps vertébraux (23, 25).

2. Cage de fusion intersomatique selon la revendication 1, caractérisée en ce que le bec de canard comprend une lèvre commune (6) réunissant deux échancrures (8, 9) formées dans deux faces d'appui opposées (4, 5) de la cage.

3. Cage de fusion intersomatique selon la revendication 2, caractérisée en ce que le bec de canard s'étend sur une fraction de longueur (I) comprise entre un cinquième et un quart de la longueur (L) totale de la cage.

4. Cage de fusion intersomatique selon la revendication 2, caractérisée en ce que la lèvre (6) possède une hauteur (h) qui est comprise entre un quart et un tiers de la hauteur (H) entre les faces d'appui opposées (4, 5).

5. Cage de fusion intersomatique selon la revendication 2, caractérisée en ce la lèvre (6) possède des angles (13, 14) cassés en biseau.

6. Cage de fusion intersomatique selon la revendication 1, caractérisée en ce que les faces d'appui opposées (4, 5) possèdent en surface un motif de rugosité (21).

7. Cage de fusion intersomatique selon la revendication 1, caractérisée en ce que l'extrémité (7) sur laquelle s'exercent les impacts lors de l'insertion comprend une prise (24) de fixation d'un outil d'impaction.

8. Cage de fusion intersomatique selon la revendication 7, caractérisée en ce que la prise de fixation est un trou taraudé (24) prévu pour recevoir une tige filetée.

9. Cage de fusion intersomatique selon la revendication 8, caractérisée en ce que le trou taraudé (24) est un trou borgne.
